(19) Europäisches Patentamt
European Patent Office
Office européen des brevets

(11) **EP 4 703 379 A1**

(12) **EUROPEAN PATENT APPLICATION**
published in accordance with Art. 153(4) EPC

(43) Date of publication:
**04.03.2026 Bulletin 2026/10**

(21) Application number: **24796263.2**

(22) Date of filing: **26.04.2024**

(51) International Patent Classification (IPC):
*C07K 16/24* (2006.01)     *C12N 15/13* (2006.01)
*C12N 15/63* (2006.01)     *A61K 39/395* (2006.01)
*A61P 37/00* (2006.01)     *G01N 33/53* (2006.01)
*A61P 37/02* (2006.01)     *C07K 16/28* (2006.01)

(52) Cooperative Patent Classification (CPC):
**A61K 39/395; A61P 37/00; A61P 37/02;
C07K 16/00; C07K 16/24; C07K 16/28;
C12N 15/63; G01N 33/53**

(86) International application number:
**PCT/CN2024/090113**

(87) International publication number:
**WO 2024/222891 (31.10.2024 Gazette 2024/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **26.04.2023   CN 202310463873**

(71) Applicant: **Innovent Biologics (Suzhou) Co., Ltd.
Suzhou, Jiangsu 215123 (CN)**

(72) Inventors:
• **YAN, Shuling**
  **Suzhou, Jiangsu 215123 (CN)**
• **ZHENG, Shirui**
  **Suzhou, Jiangsu 215123 (CN)**
• **CAI, Chengxing**
  **Suzhou, Jiangsu 215123 (CN)**
• **QIAN, Lei**
  **Suzhou, Jiangsu 215123 (CN)**

(74) Representative: **Sagittarius IP
Marlow International
Parkway
Marlow SL7 1YL (GB)**

Remarks:
The complete document including Reference Table(s) and the Sequence Listing(s) can be downloaded from the EPO website

(54) **METHOD FOR TREATING PSORIASIS WITH RECOMBINANT INTERLEUKIN 23P19 ANTIBODY**

(57)    The present invention relates to a method for preventing or treating psoriasis in a subject, comprising administering an IL-23p19 antibody to the subject, wherein a first dose of the IL-23p19 antibody is administered at Weeks 0, 4, and 8, respectively, followed by administration of a second dose of the IL-23p19 antibody every 8 Weeks to 20 weeks; and wherein the first dose is 200 mg, and the second dose is 50 mg to 250 mg, and preferably 100 mg to 200 mg.

## Description

## TECHNICAL FIELD

The present invention relates to the field of treating diseases. More particularly, the present invention relates to a method for treating psoriasis with an interleukin-23p19 antibody (IL-23p19 antibody).

## BACKGROUND ART

Psoriasis is an immune-mediated, chronic, recurrent, inflammatory, systemic disease that is influenced by genetic and environmental factors, which may occur in all age without gender preference. The typical clinical manifestations of psoriasis include scaly erythema or plaques which are localized or wide distribution, with no infections, difficult to treat and often life-long. Psoriasis can be classified into psoriasis vulgaris (including guttate psoriasis and plaque psoriasis), pustular psoriasis, erythrodermic psoriasis, and arthritic psoriasis.

At present, the global prevalence of psoriasis is approximately 2%, and 80%-90% of patients suffer from plaque psoriasis, with nearly one-third of the cases are moderate-to-severe. The prevalence rate of psoriasis varies significantly across different regions around the world. In China, it was reported that the prevalence rate of psoriasis was 0.123% in 1984, and a survey in 2008 across six cities in China showed the prevalence rate is 0.47%. Based on this, it is estimated that there are more than approximately 6 million psoriasis patients in China.

Currently, in China, the main systemic therapeutic drugs include methotrexate (MTX), cyclosporine A, retinoids, and biological agents. In recent years, monoclonal antibody biological agents targeting inflammatory cytokine have been successively used for the treatment of severe psoriasis which has poor response to traditional systemic drug therapies, substantial impact on quality of life and obvious joint symptoms. These biological agents include tumor necrosis factor $\alpha$ (TNF-$\alpha$) antagonists (etanercept, infliximab, adalimumab); as well as IL-12/23 antagonists (ustekinumab) and IL-17A antagonists (secukinumab), etc. Guselkumab (Johnson & Johnson), risankizumab (AbbVie), and tildrakizumab (Merck & Co.) are humanized IgG1 monoclonal antibodies that bind to IL-23p19 subunits, and have been developed in recent years. They have demonstrated significant efficacy in the treatment of plaque psoriasis, and have been approved in many countries for the treatment of moderate-to-severe plaque psoriasis. In December 2019, guselkumab was approved in China for the treatment of adult patients with moderate-to-severe plaque psoriasis, who are suitable for systemic therapy.

Since the launch of IL-17 and IL-23p19 monoclonal antibody drugs, the treatment of psoriasis has been revolutionized. Due to its advantages in long-term efficacy, safety, and ease of use, IL-23p19 monoclonal antibodies have been made one of the best choices for biologics in the treatment of psoriasis. However, biologics are under-utilised among Chinese psoriasis patients, and there is still a huge demand for Chinese domestic biological agents that are effective, safe and have low treatment cost.

## SUMMARY OF THE INVENTION

The present invention provides a method for treating psoriasis with an IL-23p19 antibody to meet the above needs. The method of the present invention can significantly improve skin lesions in patients, with good overall safety and tolerability, as well as excellent patient compliance.

In the first aspect, the present invention provides a dosing regimen for administering an IL-23p19 antibody to a subject in need of prevention or treatment of psoriasis. The dosing regimen comprises: administering a first dose of the IL-23p19 antibody at Weeks 0, 4, and 8, respectively, followed by administration of a second dose of the IL-23p19 antibody every 8 weeks to 20 weeks (preferably every 8 weeks to 16 weeks, more preferably every 10 weeks to 14 weeks, even more preferably every 11 weeks to 13 weeks, for example every 12 weeks), wherein the first dose is 200 mg, and wherein the IL-23p19 antibody comprises the following six CDRs:

- a heavy chain VH CDR1 comprising or consisting of GYTFTSYLMH (SEQ ID NO: 1);

- a heavy chain VH CDR2 comprising or consisting of YINPYNEGTN (SEQ ID NO: 2);

- a heavy chain VH CDR3 comprising or consisting of NWDLPY (SEQ ID NO: 3);

- a light chain VL CDR1 comprising or consisting of RASQSISDYLH (SEQ ID NO: 4);

- a light chain VL CDR2 comprising or consisting of YASQSMS (SEQ ID NO: 5); and

- a light chain VL CDR3 comprising or consisting of QQGHSFPFT (SEQ ID NO: 6),

and wherein the boundaries of CDRs were determined by, for example, the AbM scheme.

In the second aspect, the present invention provides a method for preventing or treating psoriasis in a subject. The method comprises administering an IL-23p19 antibody to the subject according to the following dosing regimen: administering a first dose of the IL-23p19 antibody at Weeks 0, 4, and 8, respectively, followed by administration of a second dose of the IL-23p19 antibody every 8 weeks to 20 weeks (preferably every 8 weeks to 16 weeks, more preferably every 10 weeks to 14 weeks, even more preferably every 11 weeks to 13 weeks, for example every 12 weeks), wherein the first dose is 200 mg, and wherein the IL-23p19 antibody comprises the following six CDRs:

- a heavy chain VH CDR1 comprising or consisting of GYTFTSYLMH (SEQ ID NO: 1);

- a heavy chain VH CDR2 comprising or consisting of YINPYNEGTN (SEQ ID NO: 2);

- a heavy chain VH CDR3 comprising or consisting of NWDLPY (SEQ ID NO: 3);

- a light chain VL CDR1 comprising or consisting of RASQSISDYLH (SEQ ID NO: 4);

- a light chain VL CDR2 comprising or consisting of YASQSMS (SEQ ID NO: 5); and

- a light chain VL CDR3 comprising or consisting of QQGHSFPFT (SEQ ID NO: 6),

and wherein the boundaries of CDRs were determined by, for example, the AbM scheme.

In the third aspect, the present invention provides an IL-23p19 antibody for preventing or treating psoriasis in a subject, wherein the IL-23p19 antibody is administered to the subject according to the following dosing regimen: administering a first dose of the IL-23p19 antibody at Weeks 0, 4, and 8, respectively, followed by administration of a second dose of the IL-23p19 antibody every 8 weeks to 20 weeks (preferably every 8 weeks to 16 weeks, more preferably every 10 weeks to 14 weeks, even more preferably every 11 weeks to 13 weeks, for example every 12 weeks), wherein the first dose is 200 mg,

wherein the IL-23p19 antibody comprises the following six CDRs:

- a heavy chain VH CDR1 comprising or consisting of GYTFTSYLMH (SEQ ID NO: 1);

- a heavy chain VH CDR2 comprising or consisting of YINPYNEGTN (SEQ ID NO: 2);

- a heavy chain VH CDR3 comprising or consisting of NWDLPY (SEQ ID NO: 3);

- a light chain VL CDR1 comprising or consisting of RASQSISDYLH (SEQ ID NO: 4);

- a light chain VL CDR2 comprising or consisting of YASQSMS (SEQ ID NO: 5); and

- a light chain VL CDR3 comprising or consisting of QQGHSFPFT (SEQ ID NO: 6),

and wherein the boundaries of CDRs were determined by, for example, the AbM scheme.

In the fourth aspect, the present invention provides a unit dosage form comprising the IL-23p19 antibody described herein, for use in prevention or treatment of psoriasis by administering the IL-23p19 antibody according to the dosing regimens of the present invention.

In the fifth aspect, the present invention provides a kit comprising the IL-23p19 antibody described herein and a package insert printed with instructions for use of the IL-23p19 antibody in prevention or treatment of psoriasis by administering the IL-23p19 antibody according to the dosing regimens of the present invention.

In all aspects of the present invention described above, administering a second dose every 8 weeks to 20 weeks, for example every 12 weeks, during the maintenance period can improve patient compliance to the medication, reduce their fear of injections, and alleviate their physical and psychological burdens, thereby helping to maintain the long-term remission in the patient.

Other embodiments of the present invention will become apparent by referring the detailed description hereafter.

## DETAILED DESCRIPTION OF THE INVENTION

Before the present invention is described in detail, it should be understood that the present invention is not limited to the specific methods and experimental conditions described in the present specification, since such methods and conditions may vary. In addition, the terms as used herein are for the purpose of illustrating particular embodiments only, and are not intended to be limiting.

### Definition

Unless otherwise defined, all technical and scientific terms as used herein have the same meaning as commonly understood by those of ordinary skill in the art. For the purposes of the present invention, the following terms are defined below.

The term "approximately", when used in conjunction with a numerical value, is intended to encompass the numerical values within a range having a lower limit of 5% less than the specified numerical value and an upper limit of 5% greater than the specified numerical value. In short, the term "approximately" means $\pm$ 5% of the numerical value with which it conjunction with. The term "and/or", when used to connect two or more options, should be understood to mean any one of the options or any two or more of the options.

The term "comprise" or the variants thereof such as "comprises" and "comprising", or "include" or the variants thereof such as "includes" and "including" as used herein means the inclusion of the stated elements, values, or steps, but does not exclude any other elements, values, or steps. When the term "include" or the variants thereof such as "comprises" and "comprising" or "comprise" or the variants thereof such as "includes" and "including" is used herein, unless otherwise specified, it also encompasses "consist of" or "consisting of" the mentioned elements, values, or steps. That is, the term "comprise" or the variants thereof such as "comprises" and "comprising", or "include"_or the variants thereof such as "includes" and "including" also encompasses "consist of" and the variants thereof such as "consisting of". For example, when referring to an antibody variable region "comprising" a specific sequence, it is also intended to encompass an antibody variable region consisting of this specific sequence.

The p19 subunit of IL-23 (also referred to herein as "IL-23p19" and "p19 subunit") is a polypeptide having 189 amino acids which comprises a 21 amino acids leader sequence (Oppmann et al., Immunity 13: 715 (2000), SEQ ID NO: 181), and four packed $\alpha$-helices designated A, B, C, and D, with an up-up-down-down topology. The four helices are linked via three polypeptide loops. The A-B and C-D loops are modeled to be relatively long as they connect parallel helices. The short B-C loop connects to antiparallel B and C helices. The p19 subunit of IL-23 is a member of the IL-6 family of helical cytokines. This family of cytokines bind to their homologous receptors via three conserved epitopes (sites I, II, and III; Bravo and Heath (2000) EMBO J. 19: 2399-2411). The p19 subunit interacts with three cytokine receptor subunits to form the competent signal transduction complex. When expressed in cells, the p19 subunit first forms a complex with the p40 subunit, which is shared by the p19 subunit and IL-12. The p19p40 complex is secreted from cells as a heterodimeric protein and is referred to as IL-23. In an embodiment, the IL-23p19 of the present invention is derived from human (NCBI: AAG37232) or cynomolgus monkey (NCBI: AEY84629).

As used herein, the term "anti-IL-23p19 antibody", "anti-IL-23p19", "IL-23p19 antibody", or "antibody binding to IL-23p19" or "antibody that binds to IL-23p19" are used interchangeably and refer to an antibody capable of binding to the (human or cynomolgus monkey) IL-23p19 subunit or a fragment thereof with sufficient affinity, such that the antibody can be used as a diagnostic and/or therapeutic agent targeting (human or cynomolgus monkey) IL-23p19.

As used herein, the term "antibody" is used in the broadest sense and refers to a protein comprising an antigen-binding site, and encompasses natural antibodies and artificial antibodies with various structures, including but not limited to full-length antibodies, antigen-binding fragments of antibodies, or multispecific antibodies, for example, bispecific antibodies.

As used herein, the terms "whole antibody", "full-length antibody", "complete antibody", and "intact antibody" are used interchangeably herein and refer to glycoproteins comprising at least two heavy chains (H) and two light chains (L) interconnected by disulfide bonds. Each heavy chain consists of heavy chain variable regions (abbreviated as VH herein) and heavy chain constant regions. The heavy chain constant region consists of three domains CH1, CH2 and CH3. Each light chain consists of light chain variable regions (abbreviated as VL herein) and light chain constant regions. The light chain constant region consists of one domain CL. The VH and VL regions can be further subdivided into regions of hypervariability, also known as complementary determining regions (CDRs), interspersed with regions that are more conserved, also known as framework regions (FRs). Each VH or VL consists of three CDRs and four FRs, which are arranged from amino-terminus to carboxy-terminus in the following order: FR1, CDR1, FR2, CDR2, FR3, CDR3, and FR4. The constant region is not directly involved in the binding an antibody to an antigen, but exhibiting many effector functions.

As used herein, the term "complementarity determining region" or "CDR region" or "CDR" can be used interchangeably, and refers to a region in an antibody variable domain that is highly variable in sequence and forms a structurally defined loop ("hypervariable loop") and/or comprises antigen-contacting residues ("antigen contact sites").

CDRs are primarily responsible for binding to an antigen epitope. The CDRs of the heavy and light chains are generally referred to as CDR1, CDR2 and CDR3, which are numbered in sequence from N-terminus. The CDRs located in heavy chain variable domains of an antibody are referred to as HCDR1, HCDR2, and HCDR3, and the CDRs located in light chain variable domains of an antibody are referred to as LCDR1, LCDR2, and LCDR3.

In a given amino acid sequence of light chain variable regions or heavy chain variable regions, the precise amino acid sequence boundary of each CDR can be determined using any one or a combination of many well-known antibody DCR numbering systems including, e.g., Chothia based on on the three-dimensional structure of antibodies and the topology of CDR loops (Chothia et al, (1989) Nature 342: 877-883; Al-Lazikani et al, "Standard conformations for the canonical structures of immunoglobulins", Journal of Molecular Biology, 273, 927-948 (1997)), Kabat based on the variability of antibody sequences (Kabat et al, Sequences of Proteins of Immunological Interest, 4th edition, U.S. Department of Health and Human Services, National Institutes of Health (1987)), AbM (University of Bath), Contact (University College London), International ImMunoGeneTics database (IMGT) (World Wide Web imgt.cines.fr/), and North CDR definitions based on affinity propagation clustering using a large amount of crystal structures.

Exemplary AbM scheme are as follows.

| CDR | AbM protocol |
| --- | --- |
| LCDR1 | L24-L34 |
| LCDR2 | L50-L56 |
| LCDR3 | L89-L97 |
| HCDR1 | H26-H35B |
| HCDR2 | H50-H58 |
| HCDR3 | H95-H102 |

Unless otherwise specified, when referring to residue positions of an antibody variable region (including heavy chain variable region residues and light chain variable region residues in) in the present invention, refer to positions numbered based on the Kabat numbering system (Kabat et al, Sequences of Proteins of Immunological Interest, 5th Ed. Public Health Service, National Institutes of Health, Bethesda, Md. (1991)).

In an embodiment, the boundaries of CDRs of the antibody of the present invention were determined by the AbM scheme.

As used herein, the term "antigen-binding fragment" refers to a molecule different from an intact antibody, which comprises a portion of the intact antibody and binds to the antigen to which the intact antibody binds. Examples of the antigen-binding fragment include, but are not limited to, Fv, Fab, Fab', Fab'-SH, F(ab')2; diabodies; linear antibodies; single-chain antibodies (e.g., scFv); single-domain antibodies; bivalent or bispecific antibodies or fragments thereof; Camelidae antibodies; and bispecific or multispecific antibodies formed from antibody fragments.

As used herein, the term "psoriasis" refers to a non-infectious disease with an unclear etiology, it is generally believed that the immune system plays a role in the onset of psoriasis. Psoriasis has a genetic predisposition and can be triggered by external factors such as infections. Psoriasis includes, but is not limited to, psoriasis vulgaris (including guttate psoriasis and plaque psoriasis), pustular psoriasis, erythrodermic psoriasis, and arthritic psoriasis (e.g., psoriatic arthritis). The psoriasis described herein can be of any severity, for example, mild, moderate, or severe psoriasis, with the moderate and severe psoriasis ("moderate-to-severe psoriasis") has a higher risk of complications, for example, ischemic heart disease, stroke, hypertension, dyslipidemia, diabetes, and Crohn's disease. Preferably, the psoriasis described herein is moderate-to-severe psoriasis, for example, moderate-to-severe plaque psoriasis. The psoriasis often manifests as one or more red, silvery, shiny plaques localized on the scalp, elbows, knees, back, or buttocks. The skin around the eyebrows, axillae, umbilicus, and perianal region, as well as the skin at the connection between the buttocks and lower back, may also be affected. The psoriasis can also manifest as deformed, thickened, or pitted nails. Depending on the affected area, the psoriasis can also be classified as, for example, scalp psoriasis, nail psoriasis, palmoplantar psoriasis, genital psoriasis, etc.

As used here, the term "unit dosage form" refers to a dosage form containing a certain amount of IL-23p19 antibody to be administered to a patient at the time of administration. Preferably, the unit dosage form described herein is used for parenteral administration, and is contained in, for example, vials for injection, ampoules, or prefilled syringes, in which a solution or lyophilized powder comprising IL-23p19 antibody is contained.

As used herein, the term "lyophilized powder" refers to powder prepared by freeze-drying, which is used for immediate administration to a subject after dilution with a suitable injectable vehicle (e.g., water for injection, sodium chloride injection, glucose injection, etc.).

As used herein, the term "parenteral administration" means a mode of administration other than enteral and topical administration, for example, injection or infusion, including but not limited to intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural, and intrasternal injection and infusion.

As used herein, the term "subject" refers to an animal. Preferably, the subject refers to a mammal, for example, primates (e.g., humans, monkeys, and gorillas), cattle, sheep, goats, horses, dogs, cats, rabbits, rats, mice and the like. More preferably, the subject is a human.

As used herein, the term "Week 0" refers to the time point of the first administration of the IL-23p19 antibody. The term "Week 4" as used herein refers to four weeks after the time point of the first administration of the IL-23p19 antibody. The term "Week 8" as used herein refers to eight weeks after the time point of the first administration of the IL-23p19 antibody. Other similar terms should be understood accordingly.

As used herein, the term "treatment" or "treating" refers to alleviating or curing psoriasis or its symptoms, and slowing or stopping the progression of psoriasis or its symptoms.

As used herein, the term "prevention" or "preventing" refers to delaying or preventing the onset of psoriasis or its symptoms.

As used herein, the term "first dose" is 200 mg of the IL-23p19 antibody described herein, and the first dose may be administered by administering one or more unit dosage forms to a subject.

As used herein, the term "second dose" is 50 mg to 250 mg, preferably 100 mg to 200 mg, for example, 100 mg, 150 mg, or 200 mg of the IL-23p19 antibody described herein, and the second dose may be administered by administering one or more unit dosage forms to a subject.

As used herein, the term "PASI" refers to psoriasis area and severity index. PASI is an established tool for measuring the efficacy of medicaments for psoriasis, and can be used to provide a numerical score (ranging from 0 to 72) for all conditions of psoriasis. Higher PASI indicates more severe psoriasis.

As used herein, the term "sPGA" refers to static Physician's Global Assessment, which is used to evaluate the psoriasis skin lesions of a subject at a given time point, with common scoring criteria for sPGA are 4-point or 5-point scoring criteria. In a case of the 4-point scoring criteria, the specific scoring criteria includes: 0 = clear; 1 = minimal; 2 = mild; 3 = moderate; 4 = severe. In a case of the 5-point scoring criteria, the specific scoring criteria includes: 0 = clear; 1 = minimal; 2 = mild; 3 = moderate; 4 = marked; 5 = severe. Higher sPGA score indicates more severe psoriasis skin lesions.

As used herein, the term "IGA" refers to investigator global assessment. Similar to sPGA6, IGA is also used to assess the psoriasis skin lesions of a subject at a given time point, with common scoring scales being the 4-point or 5-point scoring criteria. In a case of the 4-point scoring criteria, the specific scoring criteria includes: 0 = clear; 1 = minimal; 2 = mild; 3 = moderate; 4 = severe. In a case of the 5-point scoring criteria, the specific scoring criteria includes: 0 = clear; 1 = minimal; 2 = mild; 3 = moderate; 4 = marked; 5 = severe. Higher IGA score indicates more severe psoriasis skin lesions.

**Dosing regimens and treatment methods**

In the first aspect, the present invention provides a dosing regimen for administering an IL-23p19 antibody to a subject in need of prevention or treatment of psoriasis. The dosing regimen comprises: administering a first dose of the IL-23p19 antibody at Weeks 0, 4, and 8, respectively, followed by administration of a second dose of the IL-23p19 antibody every 8 weeks to 20 weeks (preferably every 8 weeks to 16 weeks, more preferably every 10 weeks to 14 weeks, even more preferably every 11 weeks to 13 weeks, for example every 12 weeks), wherein the first dose is 200 mg.

In the second aspect, the present invention provides a method for preventing or treating psoriasis in a subject. The method comprises administering an IL-23p19 antibody to the subject according to the following dosing regimen: administering a first dose of the IL-23p19 antibody at Weeks 0, 4, and 8, respectively, followed by administration of a second dose of the IL-23p19 antibody every 8 weeks to 20 weeks (preferably every 8 weeks to 16 weeks, more preferably every 10 weeks to 14 weeks, even more preferably every 11 weeks to 13 weeks, for example every 12 weeks), wherein the first dose is 200 mg.

In an embodiment of the above two aspects, the IL-23p19 antibody is administered to the subject according to the following dosing regimen: administering a first dose of the IL-23p19 antibody at Weeks 0, 4, and 8, respectively, followed by administration of a second dose of the IL-23p19 antibody every 12 weeks, for example, at Weeks 20, 32, 44, 56, and 68, respectively, wherein the first dose is 200 mg.

In an embodiment of the above two aspects, the IL-23p19 antibody is administered to the subject according to the following dosing regimen: administering a first dose of the IL-23p19 antibody at Weeks 0, 4, and 8, respectively, followed by administration of a second dose of the IL-23p19 antibody every 12 weeks, for example, at Weeks 20, 32, 44, 56, and 68, respectively, wherein the first dose is 200 mg, and the second dose is 50 mg to 250 mg, preferably, 100 mg to 200 mg.

In a specific embodiment of the above two aspects, the IL-23p19 antibody is administered to the subject according to the following dosing regimen: administering a first dose of the IL-23p19 antibody at Weeks 0, 4, and 8, respectively, followed by administration of a second dose of the IL-23p19 antibody every 12 weeks, for example, at Weeks 20, 32, 44, 56, and 68, respectively, wherein the first dose is 200 mg, and the second dose is 50 mg to 250 mg, preferably, 100 mg to 200 mg, for example, 100 mg, 120 mg, 150 mg, 160 mg, 170 mg, 180 mg, 190 mg, or 200mg, more preferably, 100 mg or 200 mg.

In a specific embodiment of the above two aspects, the administering/administration is carried out by injection, preferably subcutaneous injection.

In a preferred embodiment of the above two aspects, the IL-23p19 antibody is contained in a prefilled syringe, which preferably comprises an IL-23p19 antibody solution at the concentration of 100 mg/mL or 200 mg/mL.

In a specific embodiment of the above two aspects, the psoriasis is plaque psoriasis, preferably moderate-to-severe plaque psoriasis.

The IL-23p19 antibody used in the dosing regimens or methods of the present invention is an antibody that specifically binds to IL-23p19 and comprises the following six CDRs:

- a heavy chain VH CDR1 comprising or consisting of GYTFTSYLMH (SEQ ID NO: 1);

- a heavy chain VH CDR2 comprising or consisting of YINPYNEGTN (SEQ ID NO: 2);

- a heavy chain VH CDR3 comprising or consisting of NWDLPY (SEQ ID NO: 3);

- a light chain VL CDR1 comprising or consisting of RASQSISDYLH (SEQ ID NO: 4);

- a light chain VL CDR2 comprising or consisting of YASQSMS (SEQ ID NO: 5); and

- a light chain VL CDR3 comprising or consisting of QQGHSFPFT (SEQ ID NO: 6),

and wherein the boundaries of CDRs were determined by, for example, the AbM scheme.

In an embodiment, the heavy chain of the IL-23p19 antibody used in the dosing regimens or methods of the present invention comprises an N-glycosylation site, which is asparagine at position 295 of the heavy chain. Thus, in an embodiment, the IL-23p19 antibody used in the dosing regimens or methods of the present invention is a glycosylated antibody, for example, an antibody glycosylated at asparagine at position 295.

In an embodiment, the IL-23p19 antibody used in the dosing regimens or methods of the present invention comprises a heavy chain variable region VH and/or a light chain variable region VL, wherein the heavy chain variable region comprises or consists of the sequence as set forth in SEQ ID NO: 7 or a sequence having at least 90%, 95%, 98%, or 99% identity thereto, and the light chain variable region comprises or consists of the sequence as set forth in SEQ ID NO: 8 or a sequence having at least 90%, 95%, 98%, or 99% identity thereto:

Sequence (SEQ ID NO: 7)

QVQLVQSGAEVKKPGASVKVSCKASGYTFTSYLMHWVRQAPGQGLEWMGYINPYNEGT
NYAQKFQGRVTMTRDTSISTAYMELSRLRSDDTAVYYCARNWDLPYWGQGTLVTVSS;

Sequence (SEQ ID NO: 8)

DIQMTQSPSSLSASVGDRVTITCRASQSISDYLHWYQQKPGKAPKLLIKYASQSMSGVPSRF
SGSGSGSDFTLTISSLQPEDFATYYCQQGHSFPFTFGQGTKLEIK.

In an embodiment, the IL-23p19 antibody used in the dosing regimens or methods of the present invention comprises a heavy chain and/or a light chain, wherein the heavy chain comprises or consists of the sequence as set forth in SEQ ID NO: 9 or a sequence having at least 90%,95%, 98%, or 99% identity thereto, and wherein the light chain comprises or consists of the sequence as set forth in SEQ ID NO: 10 or a sequence having at least 90%, 95%, 98%, or 99% identity thereto. In an embodiment, the IL-23p19 antibody used in the dosing regimens or methods of the present invention is an IgG1-type antibody comprising the heavy chain and light chain as defined in the present invention. In an embodiment, the IL-23p19 antibody used in the dosing regimens or methods of the present invention comprises or consists of two heavy chains and two light chains as described, for example, two identical heavy chains and two identical light chains as described.

Sequence (SEQ ID NO: 9)

QVQLVQSGAEVKKPGASVKVSCKASGYTFTSYLMHWVRQAPGQGLEWMGYINPYNEGT
NYAQKFQGRVTMTRDTSISTAYMELSRLRSDDTAVYYCARNWDLPYWGQGTLVTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPSVFL
FPPKPKDTLYITREPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFS
CSVMHEALHNHYTQKSLSLSPG

Sequence (SEQ ID NO: 10)

DIQMTQSPSSLSASVGDRVTITCRASQSISDYLHWYQQKPGKAPKLLIKYASQSMSGVPSRF
SGSGSGSDFTLTISSLQPEDFATYYCQQGHSFPFTFGQGTKLEIKRTVAAPSVFIFPPSDEQLK
SGTASVVCLLNNFYPREAKVQWKVDNALQSGNSQESVTEQDSKDSTYSLSSTLTLSKADY
EKHKVYACEVTHQGLSSPVTKSFNRGEC

Preferably, the IL-23p19 antibody used in the dosing regimens and methods of the present invention is the IL-23p19 antibody 17D1-YTE disclosed in the patent application with PCT Application Number PCT/CN2019/121261 (International Filing Date: November 27, 2019, International Publication Number: WO2020/108530A1), said antibody consists of a heavy chain consisting of the sequence as set forth in SEQ ID NO: 9 and a light chain consisting of the sequence as set forth in SEQ ID NO: 10. This patent application is incorporated herein in its entirety as its contents were fully set forth herein.

In an embodiment, the IL-23p19 antibody used in the dosing regimens or methods of the present invention comprises the heavy chain as set forth in SEQ ID NO: 9 and the light chain as set forth in SEQ ID NO: 10.

In an embodiment, the IL-23p19 antibody used in the dosing regimens or methods of the present invention is a full-length antibody. In an embodiment, the IL-23p19 antibody used in the dosing regimens and methods of the present invention further encompasses antigen-binding fragments.

In an embodiment, the IL-23p19 antibody used in the dosing regimens or methods of the present invention is expressed recombinantly in HEK 293 cells or CHO cells.

The IL-23p19 antibody used in the dosing regimens or methods of the present invention may be formulated into a formulation for administration. Preferably, the IL-23p19 antibody may be formulated according to the formula of formulation and preparation method involving the IL-23p19 antibody disclosed in the patent application with PCT Application Number PCT/CN2021/093219 (International Filing Date: May 12, 2021, International Publication Number: WO2021/228113A1). For example, the formula of the formulation comprising IL-23p19 antibody comprises the IL-23p19 antibody at the concentration of 100.0 mg/mL, 0.76 mg/mL histidine, 1.08 mg/mL histidine hydrochloride, 50.00 mg/mL sorbitol, and 0.5 mg/mL polysorbate 80, at pH $6.0 \pm 0.5$ (5.9-6.5, for example, 6.0, 6.1, 6.2, 6.3, 6.4, or 6.5), preferably pH $6.0 \pm 0.3$. This patent application is incorporated herein in its entirety as its contents were fully set forth herein.

**Unit dosage form**

A unit dosage form comprises an IL-23p19 antibody. Preferably, the unit dosage form comprises 50 mg to 500 mg, for example, 50 mg, 60 mg, 70 mg, 80 mg, 90 mg, 100 mg, 150 mg, 200 mg, 300 mg, 350 mg, 400 mg, or 500 mg, of the IL-23p19 antibody. More preferably, the unit dosage form comprises 50 mg to 200 mg, for example, 50 mg, 100 mg, or 200 mg, of the IL-23p19 antibody. The unit dosage form is for use in prevention or treatment of psoriasis by administering according to the dosing regimens of the present invention. The psoriasis is, for example, plaque psoriasis, preferably moderate-to-severe plaque psoriasis.

The unit dosage form described herein is preferably used for parenteral administration, for example for injection, preferably subcutaneous injection. The unit dosage form may be a vial for injection, an ampoule, or a prefilled syringe, which comprises a solution or lyophilized powder comprising the IL-23p19 antibody. More preferably, the unit dosage form described herein is a prefilled syringe for parenteral administration, which comprises a solution or lyophilized powder comprising 50-200 mg, for example 50 mg, 100 mg, or 200 mg, preferably 50 mg to 100 mg, of the IL-23p19 antibody.

In an embodiment, the IL-23p19 antibody is contained in a prefilled syringe, which preferably comprises an IL-23p19 antibody solution at the concentration of 100 mg/mL or 200 mg/mL.

Kit

A kit comprises an IL-23p19 antibody. Preferably, the kit comprises the unit dosage form described above, and further comprises a package insert printed with instructions for administering the IL-23p19 antibody according to the dosing regimens of the present invention in prevention or treatment of psoriasis.

The kit is for use in prevention or treatment of psoriasis by administering the IL-23p19 antibody according to the dosing regimens of the present invention. The psoriasis is, for example, plaque psoriasis, preferably moderate-to-severe plaque psoriasis.

Uses

The IL-23p19 antibody described herein can be used for preventing or treating psoriasis. Accordingly, the present invention further relates to the use of the IL-23p19 antibody in the manufacture of a medicament for preventing or treating psoriasis in a subject, wherein the IL-23p19 antibody is administered to the subject according to the dosing regimens described above. The psoriasis is, for example plaque psoriasis, preferably moderate-to-severe plaque psoriasis.

Examples

### Example 1. Preparation and Purification of IL-23p19 Antibody

According to PCT Application Number PCT/CN2019/121261, the antibody 17D1-YTE specifically binding to IL-23p19 was obtained, said antibody consists of the heavy chain consisting of the sequence as set forth in SEQ ID NO: 9 and the light chain consisting of the sequence as set forth in SEQ ID NO: 10.

Briefly, the antibody was expressed recombinantly in CHO cells, the IL-23p19 antibody sample for the pH screening experiment of the present invention was obtained by purification using affinity chromatography, and the IL-23p19 antibody sample for the formula screening experiment of the present invention was obtained by purification using cation exchange chromatography.

### Example 2. Preparation of the Formulation comprising IL-23p19 Antibody

2.1 Experimental steps

According to PCT Application Number PCT/CN2021/093219, the formulation comprising the IL-23p19 antibody used in the present invention was formulated. Specifically, 20 mM sodium citrate and 150 mM sodium chloride were formulated; the pH was adjusted with hydrochloric acid; the purified IL-23p19 antibody from Example 1 was ultrafiltered and displaced into the above buffer solutions with different pH values; the protein content was adjusted to 50 mg/ml; and the resultant was filter and aliquoted into vials, stoppered, and capped.

The obtained formula of formulation includes: 100 mg/ml recombinant anti-IL-23p19 antibody, 0.76 mg/ml histidine, 1.08 mg/ml histidine hydrochloride, 50.00 mg/ml sorbitol, and 0.50 mg/ml polysorbate 80, pH 6.0.

### Example 3. Clinical Trials

3.1 Study objectives

Primary objective:

- To evaluate the efficacy of IL-23p19 antibody by subcutaneous injection in the treatment of moderate-to-severe plaque psoriasis as compared with placebo.

Secondary objectives:

- To evaluate the safety of the IL-23p19 antibody in the treatment of moderate-to-severe plaque psoriasis;

- To evaluate the efficacy of IL-23p19 in long-term treatment of moderate-to-severe plaque psoriasis;

- To evaluate the population pharmacokinetic characteristics of IL-23p19 in patients with moderate-to-severe plaque psoriasis;

- To evaluate the immunogenicity of IL-23p19 in patients with moderate-to-severe plaque psoriasis;

- To evaluate the impact of IL-23p19 treatment on the health-related quality of life in patients with moderate-to-severe plaque psoriasis;

- To evaluate the efficacy of IL-23p19 in treating scalp psoriasis in patients with scalp psoriasis at baseline;

- To evaluate the efficacy of IL-23p19 in treating nail leision in patients with nail psoriasis at baseline;

- To evaluate the efficacy of IL-23p19 in treating palmoplantar psoriasis in patients with palmoplantar psoriasis at baseline;

- To evaluate the efficacy of IL-23p19 in treating genital psoriasis in patients with genital psoriasis at baseline; and

- To explore the efficacy of IL-23p19 on baseline PsA activity scores in patients with psoriatic arthritis (PsA).

3.2 Principle of study design

The recommended dosing regimen for the IL-23p19 antibody was based on data analysis from the single-dose study in healthy Chinese subjects (CIL-23P19 antibody A101) and the multiple-dose study in subjects with psoriasis (CIL-23P19 antibody A201) that have been carried out, specifically including: 1) clinical results for safety; 2) exposure-primary endpoint efficacy analysis; and 3) PopPK/PASI modeling and simulation. The proposed recommended dosing regimen includes: 200 mg of the IL-23P19 antibody by subcutaneous injection at Weeks 0, 4, and 8, followed by subcutaneous injection of 100 mg or 200 mg of the IL-23p19 antibody every 12 weeks.
The specific basis is as follows:
The IL-23p19 antibody exhibited good safety: in both of the single-dose study in healthy subjects and the multiple-dose study in subjects with psoriasis, the overall safety in the subjects was favorable, with no significant increase in the incidence of adverse events compared to the placebo group, and no dose-dependent safety events were observed.
Increasing the exposure of loading administration (average concentration) increased the proportion of subjects achieving the primary endpoint (PASI90 at Week 16): increasing the average concentration of the IL-23P19 antibody (Cavg,16w) within 16 weeks increased the proportion of psoriasis patients achieving PASI90 at Week 16. According to the simulation results of robust PopPK/PASI model, it is expected that 57.5% of psoriasis patients achieve PASI90 at Week 16, by subcutaneous injection of 200 mg of the IL-23P19 antibody at Weeks 0, 4, and 8.
Extended-interval (administrated every 12 weeks, Q12W) maintenance administration sustained long-term benefits in psoriasis patients: based on the simulation results of robust PopPK/PASI model, it is expected that 83% and 88% of subjects achieve long-term (Week 52) PASI90 by the use of maintenance administration of 100 mg and 200 mg Q12W, respectively; and both of these two long-term maintenance dosing regimens were anticipated to provide the most convenient treatment regimens and treatment regimens achieving stable efficacy for psoriasis patients.
Based on the above analysis, the proposed recommendation was: 200 mg of the IL-23P19 antibody by subcutaneous injection at Weeks 0, 4, and 8, followed by subcutaneous injection of 100 mg Q12W or 200 mg Q12W for the subsequent clinical studies.

3.3 Study design

This study is based on a multicenter, randomized, double-blind, placebo-controlled design, and the primary objective is to evaluate the efficacy of the IL-23P19 antibody by subcutaneous injection in the treatment of moderate-to-severe plaque psoriasis as compared to placebo. The target population consists of male or female subjects aged 18 to 75 years, who has been diagnosed with plaque psoriasis with or without psoriatic arthritis for at least 6 months prior to the first administration of the investigational agent. The subject must be a candidate who has been diagnosed with plaque psoriasis, with sPGA of ≥ 3 and a PASI of ≥ 12, the affected body surface area (BSA) ≥10%. The planned enrollment of this study is 500 patients with moderate-to-severe plaque psoriasis.
After a 4-week screening period, eligible subjects will be randomized in a 1:2:2 ratio into placebo group, IL-23P19 antibody group 1 (the administration group with an initial dose of 200 mg of the IL-23P19 antibody, followed by 200 mg every 12 weeks), or IL-23P19 antibody group 2 (the administration group with an initial dose of 200 mg of the IL-23P19 antibody, followed by 100 mg every 12 weeks). Subjects will be stratified based on the previous use of biologics for the treatment of psoriasis.

- Placebo group: subcutaneous injection of placebo at Weeks 0, 4, and 8, and subcutaneous injection of 200 mg of the IL-23P19 antibody at Weeks 16, 20, 24, 32, and 44.

- IL-23P19 antibody group 1: subcutaneous injection of 200 mg of the IL-23P19 antibody at Weeks 0, 4, and 8, followed by administration every 12 weeks, i.e., subcutaneous injection of 200 mg of the IL-23P19 antibody at Weeks 20, 32, and 44. To maintain blinding, subcutaneous injection of placebo is carried out at Weeks 16 and 24.

- IL-23P19 antibody group 2: subcutaneous injection of 200 mg of the IL-23P19 antibody at Weeks 0, 4, and 8, followed by administration every 12 weeks, i.e., subcutaneous injection of 100 mg of the IL-23P19 antibody at Weeks 20, 32, and 44. To maintain blinding, subcutaneous injection of placebo is carried out at Weeks 16 and 24.

The subjects will receive the last dose at Week 44, the follow-up visits and evaluation for double-blind treatment period will continue until Week 52. The double-blind treatment period is followed by a 16-week follow-up period, with the last follow-up at Week 68.

Inclusion criteria

The eligible subjects must meet all of the following inclusion criteria:

(1) Male or female aged 18 years to 75 years;

(2) Diagnosis of plaque psoriasis with or without psoriatic arthritis for at least 6 months;

(3) At the screening period and baseline, the body surface area (BSA) affected with plaque psoriasis $\geq$ 10%, the psoriasis area and severity index (PASI) score $\geq$ 12, and the static physician's global assessment (sPGA) score $\geq$ 3;

(4) Suitability for phototherapy and/or systemic treatment for psoriasis; and

(5) The subject is able to full understand the objectives of the study, and have a basic understanding the pharmacological effects and possible adverse reactions of the investigational agent, and voluntarily sign the informed consent in accordance with the spirit of the Declaration of Helsinki.

Exclusion criteria

(1) Diagnosis with other types of psoriasis (guttate psoriasis, pustular psoriasis, or erythrodermic psoriasis) than plaque psoriasis;

(2) Diagnosis with drug-induced psoriasis (e.g., psoriasis caused by beta-blockers, calcium channel inhibitors, etc.);

(3) Previously treated with IL-23P19 antibody or IL-23 targeted therapy;

(4) Treated with topical therapeutic agent (including but not limited to corticosteroids, vitamin D3 derivatives, retinoids, calcineurin inhibitors, keratolytics, and compound formulations, etc.) that may affect the evaluation for psoriasis within 2 weeks prior to the first dose of the investigational agent;

(5) Treated with systemic medications (including but not limited to methotrexate, cyclosporine, retinoids, azathioprine, leflunomide, mycophenolate mofetil, sulfasalazine, corticosteroids, JAK inhibitors such as tofacitinib and baricitinib, or Traditional Chinese Medicines or Chinese Patent Medicines for psoriasis) that may affect evaluation for psoriasis within 4 weeks prior to the first dose of the investigational agent;

(6) Treated with tumor necrosis factor-$\alpha$ (TNF-$\alpha$) antagonists (including but not limited to etanercept, infliximab, and adalimumab) within 3 months (or within 5 half-lives of the agent) prior to the first dose of the investigational agent;

(7) Treated with IL-17 targeted therapies (including but not limited to secukinumab, ixekizumab, etc.) within 6 months (or within 5 half-lives of the agent) prior to the first dose of the investigational agent;

(8) Treated with Natalizumab, or B-cell or T-cell modulators (e.g., rituximab, abatacept, or visilizumab) within 12 months prior to the first dose of the investigational agent;

(9) Treated with phototherapy for psoriasis within 1 month prior to the first dose of the investigational agent, and/or unwillingness to avoid prolonged sun exposure and other ultraviolet light exposure during the study;

(10) Evidence of severe, progressive, and uncontrolled (including but not limited to) cardiovascular diseases, neuromuscular diseases, hematologic diseases, respiratory diseases, hepatic or digestive diseases, urinary diseases, neurological or psychiatric diseases in the subject;

(11) Had opportunistic infections (for example, herpes zoster (severe or recurrent), infections with active cytomegalovirus, Pneumocystis carinii, Histoplasma, Aspergillus, Mycobacterium, etc.) within 6 months prior to the screening period;

(12) Known history of relapse or chronic infections, or previously suffered from chronic or recurrent infections, including but not limited to: chronic kidney infections, chronic chest infections (e.g., bronchiectasis), recurrent urinary tract infections, and open, draining or skin infected wounds;

(13) History of severe infections (e.g., sepsis, pneumonia, pyelonephritis), or hospitalization or treatment with intravenous antibiotic due to infection within 2 months prior to the screening period;

(14) Has malignancy or history of malignancy (except for squamous cell carcinoma of the skin, basal cell carcinoma, or cervix carcinoma in situ that have been successfully excised, with no evidence of recurrence or metastasis within 5 years);

(15) Has or has previously suffered lymphoproliferative diseases, or presence of symptoms or signs suggesting lymphoproliferative diseases (e.g., lymphadenopathy and/or splenomegaly) within 5 years prior to the screening period;

(16) Known history of active tuberculosis or has clinical manifestations suspected of tuberculosis (including but not limited to pulmonary tuberculosis, lymphatic tuberculosis, tuberculous pleurisy, etc.), where the subject is subjected to interferon gamma release assay (IGRA) and chest X-ray (posteroanterior and lateral films) for tuberculosis screening, and for subjects without symptom of active tuberculosis or radiological evidence of tuberculosis:

- if the IGRA result is negative, the subject is eligible for enrollment,

- if the IGRA result is indeterminate, test may be re-performed, and the subject with persistently indeterminate results is ineligible for enrollment, and

- if the IGRA result is positive, the subject may be subjected to screening evaluation again after receiving at least 1 month of prophylactic anti-tuberculosis treatment, the subject without tuberculosis symptoms and with good tolerance to anti-tuberculosis drugs, who is willing to receive complete prophylactic anti-tuberculosis treatment during the study, is eligible for enrollment upon investigator assessment;

(17) Recieved Bacillus Calmette-Guérin (BCG) vaccine within 12 months prior to the first dose of the investigational agent, or has a plan to receive BCG vaccine during the study or within 12 months after the last investigational treatment;

(18) Recieved live or bacterial vaccine within 3 months prior to the first dose of the investigational agent, or has a plan to receive live or bacterial vaccine during the study or within 3 months after the last investigational treatment;

(19) Treated with an investigational biologic agent within 6 months prior to the first dose of the investigational agent, or treated with any experimental treatment within 30 days or within 5 half-lives of the investigational agent , or current participation in a clinical study;

(20) At screening period and baseline, the results of routine blood tests and blood biochemical examinations meet the following conditions:

- any indicator of hemoglobin, red blood cells, white blood cells, neutrophils, or platelets is below the lower limit of normal value (LLN), with clinically significant abnormality determined by the investigator,

- any indicator of alanine transaminase (ALT), aspartate amino transferase (AST), total bilirubin (TBIL), or direct bilirubin (DBIL) is above 2 times higher than the upper limit of normal value (ULN),

- creatinine (Cr) is greater than ULN, and

if the indicators fall within the range required by the protocol upon re-examination, the subject is also eligible for enrollment;

(21) At screening period, the viral test results meet any of the following criterias:

- Human Immunodeficiency Virus (HIV) antibody is positive,

- Hepatitis C Virus (HCV) antibody is positive, without a history of successful treatment, where the successful treatment is defined as HCV RNA negative after the completion of the antiviral therapy for at least 24 weeks,

- Hepatitis B Virus (HBV) screening includes at least Hepatitis B Surface Antigen (HBsAg), Hepatitis B Surface Antibody (HBsAb), and Hepatitis B Core Antibody (HBcAb), and if the test results for these three indicators show: HBsAg positive; or when HBsAg negative, and only HBcAb positive, then HBV DNA teste is nessesary and the HBV DNA result must be positive;

- Syphilis-specific antibody is positive (except for those who has negative non-specific antibody titer upon standard syphilis treatment);

- At screening period, with clinically significant abnormalities in 12-lead electrocardiograms (ECGs): QTcF > 450 ms, shortened or prolonged PR interval, second-degree or third-degree atrioventricular block, pre-excitation syndrome and long QT syndrome, or severe arrhythmia requiring treatment;

(22) History of severe allergic reactions to drug or food, and/or with hypersensitivity to the investigational agent or other components;

(23) History of alcohol or drug abuse within 12 months prior to the screening period;

(24) Pregnant or lactating female subjects, or reproductive-aged female with positive pregnancy test at screening period or prior to administration;

(25) Has plan to conceive during the study or within 6 months after administration of the investigational agent, or unwilling to taken effective contraceptive measures approved by the investigator (e.g., condoms) during the study; and

(26) According to the investigator's judgment, various reasons make the subject unsuitable to participate in this study.

3.4 Definition of study completion

A subject is considerd to have completed the study upon the completion of the visit at Week 68 of the study.
Premature termination of treatment/study for any reason before study completion is considered as incomplete study.
The end of the study is defined as the completion of the follow-up visit at Week 68 for the last subject.

3.4.1 Clinical criteria for discontinuation/early termination of the study

If there are sufficient and reasonable grounds, this study may be temporarily suspended or terminated prematurely. The party suspending or terminating the study shall provide the subjects, investigators, sponsors, and regulatory authorities a written notice, in which the reasons for the suspension or termination are described. If the study is terminated prematurely or suspended, the principal investigator shall promptly notify the subjects and the Ethics Committee (EC), and provide the reasons for the termination or suspension. If applicable, the investigator shall contact the subjects and notify them of changes to the scheduled visit time.

Circumstances where the termination or suspension of the study is required include, but are not limited to:

- identification of unexpected, significant, or unacceptable risks to subjects;

- evidence in terms of efficacy indicating that the study should be terminated/suspended;

- fail to meet requirements of protocol compliance;

- incomplete and/or insufficient data for evaluation; and

- has plan to change or discontinue the development of the investigational agent.

The study can proceed only when issues related to safety, protocol compliance and data quality have been resolved, and the requirements of the EC and/or the National Medical Products Administration have been met. If the sponsor decides to discontinue to supply the investigational agent, sufficient notice shall be given to allow for appropriate adjustments to the treatment for subjects.

3.5 Indexes related to Efficacy evaluation:

3.5.1 PASI

The Psoriasis Area and Severity Index (PASI) is a well-established tool for measuring the efficacy of medicaments for psoriasis. PASI can provide a numerical score ranging from 0 to 72 for all conditions of psoriasis. It can combine the percentage of affected body surface area with the severity of erythema, induration, and scales in four body regions in a linear manner. Efforts should be made to ensure that the PASI assessment for the subject at follow-up visits is performed by the physician performing the PASI assessment for the same subject at the screening period /baseline.

This endpoint is based on the percentage reduction from baseline, and is typically summarized as a binomial distribution result based on the achievement of X% reduction (or PASIx), where X can be 50, 75, 90, or 100. Therefore, PASI90 refers to a $\geq$90% improvement in PASI score from baseline; PASI75 refers to a $\geq$75% improvement in score from baseline; and PASI100 refers to a 100% improvement in score from baseline.

To calculate PASI, the areas of four main body regions are assessed: head (h), trunk (t), upper extremities (u), and lower extremities (1), which account for 10%, 30%, 20%, and 40% of the surface area of the same body, respectively.

The areas affected by psoriasis of these four regions are represented by the following number:

$$0 = \text{No affection;}$$

$$1 = {<}10\%;$$

$$2 = 10\% \text{ - } {<}30\%;$$

$$3 = 30\% \text{ - } {<}50\%;$$

$$4 = 50\% \text{ - } {<}70\%;$$

$$5 = 70\% \text{ - } {<}90\%;$$

$$6 = 90\%\text{-}100\%$$

The markers of the severity: erythema (E), induration (I), and scaling (S) of the skin lesions are assessed on a scale of 0-4, where 0 denotes skin is not affected, 1 denotes mild, 2 denotes moderate, 3 denotes severe, and 4 denotes very severe; and the scores for each of the four regions are listed separately.

To assist in area assessment, it should be noted that:

a. the neck is considered as a part of the head;

b. the axillae and groin are considered as a part of the trunk; and

c. the buttocks are considered as a part the lower extremities.

$$PASI = 0.1*(Eh+Ih+Sh)Ah + 0.3*(Et+It+St)At + 0.2*(Eu+Iu+Su)Au + 0.4*(El+Il+Sl)Al$$

3.5.2 sPGA

The static Physician's Global Assessment (sPGA) records the physician's evaluation on the psoriasis status of the subject, including the following aspects: induration, scaling, and erythema, the sPGA score is obtained through dividing the total score of 3 items by 3.
Induration (I) (average value for all skin lesions; using National Psoriasis Foundation (NPF) Reference card for measurement)

0 = No evidence of plaque elevation

1 = Minimal plaque elevation, 0.25 mm

2 = Mild plaque elevation, 0.5 mm

3 = Moderate plaque elevation, 0.75 mm

4 = Marked plaque elevation, 1 mm

5 = Severe plaque elevation, $\geq$ 1.25 mm

**Erythema(E) (Average value for all skin lesions)**

0 = No evidence of erythema, hyperpigmentation may be present

1 = Faint erythema

2 = Light redness

3 = Moderate redness

4 = Bright redness

5 = Dusky to deep redness

Scaling(S) (Average value for all skin lesions)

0 = No evidence of scaling

1 = Minimal scaling; occasional scaling accounting for <5% of total skin lesions

2 = Mild; fine-scale predominates

3 = Moderate; coarse-scale predominates

4 = Marked; thick, non-tenacious scale predominates

5 = Severe; very thick tenacious scale predominates

$$I + E + S = /3 = \textbf{(Overall average value)}$$

sPGA (static Physician Global Assessment) was evaluated based on the overall average score: sPGA was evaluated based on the overall average score:

0 = Clear, except for some residual hyperpigmentation;

1 = Minimal, the score of most individual skin lesions is1;

2 = Mild, the score of most individual skin lesions is 2;

3 = Moderate, the score of most individual skin lesions is3;

4 = Marked, the score of most individual skin lesions is 4; and

5 = Severe, the score of most individual skin lesions is 5.

Efforts should be made to ensure that the sPGA assessment for the subject at follow-up visits is performed by the physician performing the sPGA assessment for the same subject at screening period /baseline.
Higher sPGA score indicates more severe psoriatic skin lesions.

3.5.3 DLQI

The Dermatology Life Quality Index (DLQI) is a tool for evaluating dermatology-related life quality, which is used to assess the impact of the disease on the subject's quality of life (AY Finlay and GK Khan). It is a questionnaire containing 10 questions that is used to evaluate six different aspects of quality of life: symptoms and feelings, daily activities, leisure-time activities, work or school standards, personal relationships, and treatment. For visits involving DLQI assessment, the DLQI assessment during the study is conducted prior to all other visit processes (examinations, procedures, psoriasis assessments, adverse event collection, and concomitant medication collection, etc.).

3.5.4 PSSI

The Psoriasis Scalp Severity Index (PSSI) assessment is only performed for subjects with scalp psoriasis at baseline. The PSSI records thearea of scalp psoriasis, as well as the induration, scaling, and erythema of the scalp psoriatic lesions. Similar to PASI, the numerical score of PSSI ranges from 0 to 72. Efforts should be made to ensure that the PSSI assessment for the subject at follow-up visits is performed by the physician performing the PSSI assessment for the same subject at the screening period /baseline.

3.5.5 NAPSI

The Nail Psoriasis Severity Index (NAPSI) assessment is only performed for subjects with concomitant nail psoriasis at baseline. The NAPSI evaluates damage to the nail matrix and nail bed, with a total score ranging from 0 to 80. Efforts should be made to ensure that the NAPSI assessment for the subject at follow-up visits is performed by the physician performing the NAPSI assessment for the same subject at the screening period /baseline.

3.5.6 PPASI

The Palmoplantar Psoriasis Area and Severity Index (PPASI) assessment is only performed for subjects with palmoplantar psoriasis at baseline. The PPASI assesses the palms psoriasis and the soles psoriasis, with a total score ranging from 0 to 72. Efforts should be made to ensure that the PPASI assessment for the subject at follow-up visits is performed by the physician performing the PPASI assessment for the same subject at the screening period /baseline.

3.5.7 sPGA-G

The static physician's global assessment of genitalia (sPGA-G) is only performed for subjects with genital psoriasis at baseline. The sPGA-G is a localized evaluation for genital psoriasis using the sPGA assessment criteria.

Efforts should be made to ensure that the sPGA-G assessment for the subject at follow-up visits is performed by the physician performing the sPGA-G assessment for the same subject at the screening period/baseline.

3.5.8 PsA disease activity

The assessment of psoriatic arthritis disease activities is only used for subjects with psoriatic arthritis at baseline, and performed by using the global assessment of disease activity for PsA. This assessment must be carried out by the physician and subject independently from each other. Efforts should be made to ensure that the PsA disease activity assessment for the subject at follow-up visits is performed by the physician performing the PsA disease activity assessment for the same subject at baseline. The Visual Analogue Scale (VAS) is used to record the assessment of psoriatic arthritis disease activities by the "subjects" and "physicians". The VAS assessment by the subject ranges from "very good (0 cm)" to "very bad (10 cm)," while the VAS assessment by physician ranges from "no arthritis activity (0 cm)" to "arthritis and its activities (10 cm)".

3.5.9 Photography of skin lesions

To obtain additional data for efficacy evaluation, photographs of the skin condition of the subject (excluding the perineal region) were taken at each efficacy assessment.

3.6 Study endpoints

At Week 16, the IL-23P19 antibody group 1 and the IL-23P19 antibody group 2 were combined and compared with the placebo group.
Primary endpoints for efficacy:

- the proportion of subjects achieving ≥ 90% improvement in PASI (PASI90) at Week 16; and

- the proportion of subjects achieving sPGA clear (0 point) or minimal (1 point) at Week 16.

Key secondary endpoints for efficacy:

- the proportion of subjects achieving ≥ 75% improvement in PASI (PASI75) at Week 16;

- the proportion of subjects achieving 100% improvement in PASI (PASI 100) at Week 16;

- the proportion of subjects achieving sPGA clear (0 point) at Week 16;

- the proportion of subjects achieving the QLQI score of 0/1 point at Week 16;

Other secondary efficacy endpoints:

- the proportion of subjects achieving ≥ 90% improvement in PASI (PASI90) at Week 52;

- the proportion of subjects achieving ≥ 75% improvement in PASI (PASI75) at Week 52;

- the proportion of subjects achieving 100% improvement in PSAI (PASI100) at Week 52;

- the proportion of subjects achieving sPGA clear (0 point) at Week 52;

- the proportion of subjects achieving sPGA clear (0 point) or minimal (1 point) at Week 52;

- change from baseline in DLQI at Week 52;

- the proportion of subjects achieving PASI90, PASI75, PASI100, sPGA0 or sPGA0/1 at different time points, and the proportion of subjects achieving DLQI0/1 at different time points;

- median PASI improvement at different time points;

- change from baseline in PSSI at different time points (only for subjects with scalp psoriasis at baseline);

- change from baseline in NAPSI at different time points (only for subjects with nail psoriasis at baseline);

- change from baseline in PPASI at different time points (only for subjects with palmoplantar psoriasis at baseline);

- change from baseline in sPGA-G at different time points (only for subjects with genital psoriasis at baseline); and

- change from baseline in Global Assessment of Disease Activity for PsA at different time points (only for subjects with concomitant psoriatic arthritis at baseline).

Endpoints for pharmacokinetic:

Characterization of the pharmacokinetic characteristics of IL-23p19, including but not limited to elimination half-life ($t_{1/2}$), clearance (CL), and volume of distribution (V).

Safety endpoints:

All adverse events, serious adverse events, etc. Changes in vital signs, physical examinations, laboratory tests, electrocardiograms, etc. before and after administration.

Immunogenicity evaluation:

The incidence of anti-drug antibody (ADA) and neutralizing antibody (Nab).

3.7 Clinical trial results

(1) Blind data

In this Phase III clinical study, to date, 500 subjects have been enrolled, including 100 subjects for placebo group, and 200 subjects each for IL-23P19 antibody group 1 and IL-23P19 antibody group 2. At Week 16, among the 500 subjects, 326 subjects achieved PASI90, and 390 subjects achieved sPGA0/1.
No significant or unacceptable toxic side effects were observed in the 500 enrolled subjects, and the patient compliance was good.

(2) Estimated results based on the blind data

At present, this clinical trial is still ongoing, however, the 500 enrolled subjects have completed the 16-week primary clinical endpoint. Therefore, the blind data collected from these 500 subjects have not been unblinded. However, based on the prior art and these blind data, it has been sufficiently demonstrated the efficacy, safety, and good compliance in treating moderate to severe plaque psoriasis by administering the IL-23P19 antibody according to the dosing regimen of the present invention.
According to the prior art (Kenneth B Gordon et.al., Efficacy and safety of risankizumab in moderate-to-severe plaque psoriasis (UltIMMa-1 and UltIMMa-2): results from two double-blind, randomised, placebo-controlled and ustekinumab-controlled phase 3 trials, Lancet. 2018 Aug 25; 392(10148):650-661), in the phase 3 clinical study of same-target risankizumab in subjects with moderate-to-severe plaque psoriasis (PASI $\geq$ 12 and sPGA $\geq$ 3), at Week 16, the proportion of subjects achieving PASI90 in placebo group was 4.9% or 2.0% and the proportion of subjects achieving sPGA0/1 was 7.8% or 5.1%.
Based on the highest proportion that 4.9% of subjects achieving PASI90 in placebo group, it was estimated that: in the phase III clinical trial of the present application, the number of subjects achieving PASI90 in 100 subjects of placebo group would be at most 5 (=100 * 4.9%). Among these 326 subjects achieving PASI90, 321 subjects (= 326-5) were from the two treatment groups (i.e., IL-23P19 antibody group 1 and IL-23P19 antibody group 2). Therefore, the proportion of subjects achieving PASI90 in 400 subjects of the two treatment groups was 80.3% (= 321/400).
Based on the highest proportion that 7.8% of subjects achieving sPGA0/1 in the placebo group, it was estimated that: in the phase III clinical trial of the present application, the number of subjects achieving sPGA0/1 in 100 subjects of placebo group would be 8 (=100 * 7.8%). Among these 390 subjects achieving sPGA0/1, 382 subjects (= 390 - 8) were from the two treatment groups (i.e., IL-23P19 antibody group 1 and IL-23P19 antibody group 2). Therefore, the proportion of subjects achieving sPGA0/1 in the 400 subjects of the two treatment groups was 95.5% (= 382/400).

Table 1: Comparison of the effects of the IL-23P19 antibody of the present invention with known antibodies for the treatment of psoriasis

| Drug Index | Inventive IL23p19 antibody % | Guselkumab (Johnson & Johnson) %* | Guselkumab (Johnson & Johnson) %** | Secukinumab (Novartis) %*** | Risankizumab (AbbVie) %**** |
|---|---|---|---|---|---|
| PASI90 | 80.3% | 73.3% | 70.0% | 70.0% | 75.3% |
| sPGA0/1 or IGA0/1 | 95.5% | 85.1% | 84.1% | 75.0% | 87.8% |

\* Data from Adrew Blauvelt et.al, Efficacy and safety of guselkumab, an anti-interleukin-23 monoclonal antibody, compared with adalimumab for the continuous treatment of patients with moderate to severe psoriasis: Results from the phase III, double-blinded, placebo- and active comparator-controlled VOYAGE 1 trial, *J Am Acad Dermatol.* 2017 Mar; 76(3):405-417

\*\* Data from Kristian Reich et.al, Efficacy and safety of guselkumab, an anti-interleukin-23 monoclonal antibody, compared with adalimumab for the treatment of patients with moderate to severe psoriasis with randomized withdrawal and retreatment: Results from the phase III, double-blind, placebo- and active comparator-controlled VOYAGE 2 trial, *J Am Acad Dermatol.* 2017 Mar; 76(3):418-431

\*\*\* Data from Richard G. Langley et.al, Secukinumab in Plaque Psoriasis —Results of Two Phase 3 Trials, *N Engl J Med* 2014;371:326-338

\*\*\*\* Data from Kenneth B Gordon et.al., Efficacy and safety of risankizumab in moderate-to-severe plaque psoriasis (UltIMMa-1 and UltIMMa-2): results from two double-blind, randomised, placebo-controlled and ustekinumab-controlled phase 3 trials, *Lancet.* 2018 Aug 25; 392(10148):650-661

Thus, it can be seen that, based on blind data obtained from the 500 subjects in this Phase III clinical trial, it was estimated that the proportion of subjects achieving PASI90 at Week 16 was 80.3%, and the proportion of subjects achieving sPGA0/1 at Week 16 was 95.5%. Compared to the proportions of subjects achieving PASI90 and IGA0/1 at Week 16 achieved by the same-target antibodies guselkumab (Johnson & Johnson) and risankizumab (AbbVie), the IL-23p19 antibody demonstrated better therapeutic effects.

Furthermore, the current blind results from this Phase III clinical trial also demonstrated that, when the IL-23p19 antibody is used according to the dosing regimen of the present invention for treating moderate-to-severe plaque psoriasis, no unacceptable toxic side effects were observed in the 500 subjects, indicating favorable safety.

The above describes the exemplary embodiments of the present invention. Those skilled in the art should understand that these disclosures are merely exemplary, and various other substitutions, adaptations, and modifications can be made within the scope of the present invention. Therefore, the present invention is not limited to the specific embodiments enumerated herein.

**Claims**

1. A method for preventing or treating psoriasis in a subject, comprising administering an IL-23p19 antibody to said subject according to the following dosing regimen: administering a first dose of said IL-23p19 antibody at Weeks 0, 4, and 8, respectively, followed by administration of a second dose of said IL-23p19 antibody every 8-20 weeks, wherein said IL-23p19 antibody comprises the following six CDRs:

   - a heavy chain VH CDR1 comprising or consisting of GYTFTSYLMH (SEQ ID NO: 1);

- a heavy chain VH CDR2 comprising or consisting of YINPYNEGTN (SEQ ID NO: 2);
- a heavy chain VH CDR3 comprising or consisting of NWDLPY (SEQ ID NO: 3);
- a light chain VL CDR1 comprising or consisting of RASQSISDYLH (SEQ ID NO: 4);
- a light chain VL CDR2 comprising or consisting of YASQSMS (SEQ ID NO: 5); and
- a light chain VL CDR3 comprising or consisting of QQGHSFPFT (SEQ ID NO: 6),

and wherein said first dose is 200 mg, and said second dose is 50 mg to 250 mg, preferably 100 mg to 200 mg, for example, 100 mg, 120 mg, 150 mg, 160 mg, 170 mg, 180 mg, 190 mg, or 200 mg.

2. The method according to claim 1, wherein the method comprises administering said IL-23p19 antibody to said subject according to the following dosing regimen: administering a first dose of said IL-23p19 antibody at Weeks 0, 4, and 8, respectively, followed by administration of a second dose of said IL-23p19 antibody every 8-16 weeks, preferably every 10-14 weeks, and more preferably every 11-13 weeks.

3. The method according to claim 1 or 2, wherein the method comprises administering said IL-23p19 antibody to said subject according to the following dosing regimen: administering a first dose of said IL-23p19 antibody at Weeks 0, 4, and 8, respectively, followed by administration of a second dose of said IL-23p19 antibody every 12 weeks.

4. The method according to any one of claims 1 to 3, whererin the method comprises administering said IL-23p19 antibody to said subject according to the following dosing regimen: administering a first dose of said IL-23p19 antibody at Weeks 0, 4, and 8, respectively, followed by administration of a second dose of said IL-23p19 antibody at Weeks 20, 32, and 44, respectively.

5. The method according to any one of claims 1 to 4, wherein said second dose is 100 mg or 200 mg.

6. The method according to any one of claims 1 to 5, wherein said administering/administration is carried out by injection, preferably subcutaneous injection.

7. The method according to any one of claims 1 to 6, wherein said IL-23p19 antibody is contained in a prefilled syringe, which comprises an IL-23p19 antibody solution at the concentration of 100 mg/mL or 200 mg/mL.

8. The method according to any one of claims 1 to 7, wherein said psoriasis is plaque psoriasis, preferably moderate-to-severe plaque psoriasis.

9. The method according to any one of claims 1 to 8, wherein the heavy chain of said IL-23p19 antibody comprises an N-glycosylation site, which is asparagine at position 295 of the heavy chain.

10. The method according to any one of claims 1 to 9, wherein said IL-23p19 antibody comprises a heavy chain variable region VH and a light chain variable region VL, wherein said heavy chain variable region comprises or consists of the sequence as set forth in SEQ ID NO: 7 or a sequence having at least 90%, 95%, 98%, or 99% identity thereto, and the light chain variable region comprises or consists of the sequence as set forth in SEQ ID NO: 8 or a sequence having at least 90%, 95%, 98%, or 99% identity thereto.

11. The method according to any one of claims 1 to 10, wherein said IL-23p19 antibody is an IgG1-type antibody comprising a heavy chain and a light chain, wherein said heavy chain comprises or consists of the sequence as set forth in SEQ ID NO: 9 or a sequence having at least 90%, 95%, 98%, or 99% identity thereto, and the light chain comprises or consists of the sequence as set forth in SEQ ID NO: 10 or a sequence having at least 90%,95%, 98%, or 99% identity thereto.

12. The method according to any one of claims 1 to 11, wherein said IL-23p19 antibody comprises a heavy chain as set forth in SEQ ID NO: 9 and a light chain as set forth in SEQ ID NO: 10; for example, said IL-23p19 antibody comprises or consists of two heavy chains as set forth in SEQ ID NO: 9 and two light chains as set forth in SEQ ID NO: 10.

13. The method according to any one of claims 1 to 12, wherein said IL-23p19 antibody is expressed recombinantly in HEK 293 cells or CHO cells.

14. The method according to any one of claims 1 to 13, wherein said IL-23p19 antibody is comprised in a pharmaceutical formulation comprising 100.0 mg/mL IL-23p19 antibody, 0.76 mg/mL histidine, 1.08 mg/mL histidine hydrochloride, 50.00 mg/mL sorbitol, and 0.5 mg/mL polysorbate 80, pH 6.0 ± 0.5, preferably pH 6.0 ± 0.3.

15. A unit dosage form, comprising 50 mg to 500 mg, preferably 50 mg to 200 mg, for example 50 mg to 100 mg, of an IL-23p19 antibody, wherein said unit dosage form is for use in prevention or treatment of psoriasis, preferably plaque psoriasis, and more preferably moderate-to-severe plaque psoriasis, according to the method of any one of claims 1 to 14.

16. A kit, comprising: (1) 50 mg to 500 mg, preferably 50 mg to 200 mg, for example 50 mg to 100 mg, of an IL-23p19 antibody in one or more unit dosage forms; and (2) a package insert printed with instructions for use of said anti-IL-23p19 antibody according to the method of any one of claims 1 to 14 in prevention or treatment of psoriasis, preferably plaque psoriasis, and more preferably moderate-to-severe plaque psoriasis.

17. Use of the unit dosage form of claim 15 or the kit of claim 16 in the manufacture of a medicament for preventing or treating psoriasis.

## INTERNATIONAL SEARCH REPORT

| International application No. |
| --- |
| **PCT/CN2024/090113** |

| A. | CLASSIFICATION OF SUBJECT MATTER |
| --- | --- |

C07K16/24(2006.01)i; C12N15/13(2006.01)i; C12N15/63(2006.01)i; A61K39/395(2006.01)i; A61P37/00(2006.01)i; G01N33/53(2006.01)i; A61P37/02(2006.01)i; C07K16/28(2006.01)i

According to International Patent Classification (IPC) or to both national classification and IPC

| B. | FIELDS SEARCHED |
| --- | --- |

Minimum documentation searched (classification system followed by classification symbols)

IPC: C07K C12N A61K A61P G01N

Documentation searched other than minimum documentation to the extent that such documents are included in the fields searched

Electronic data base consulted during the international search (name of data base and, where practicable, search terms used)

CNTXT; WPABS; ENTXT; USTXT; JPTXT; KRTXT; DWPI; VEN; CJFD; ISI web of Science; PUBMED; 百度学术, Baidu Scholar; 百度, Baidu; 读秀, DUXIU; 万方, WANFANG; CNKI; Genbank; EBI: ENA: 中国专利生物序列检索, China Patent Biological Sequence Search; STN: 抗体, 银屑病, 斑块, 重度, 中度, 斑块银屑病, IL-23P19, IL-23, P19, 亚基, CDRS, 组氨酸, 盐酸组氨酸, 山梨醇, 山梨酯80, antibody, psoriasis, plaque, plaque psoriasis, severe, moderate, SEQ ID NOs: 1-10

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2021228113 A1 (INNOVENT BIOLOGICS (SUZHOU) CO., LTD.) 18 November 2021 (2021-11-18) description, page 2, paragraph 3 to page 4, last paragraph, and page 6, paragraph 2 from the bottom to page 7, paragraph 4, and claims 1-16 | 1-7, 9-17 |
| Y | WO 2021228113 A1 (INNOVENT BIOLOGICS (SUZHOU) CO., LTD.) 18 November 2021 (2021-11-18) description, page 2, paragraph 3 to page 4, last paragraph, and page 6, paragraph 2 from the bottom to page 7, paragraph 4, and claims 1-16 | 8-14 |
| Y | CN 112638420 A (ELI LILLY AND CO.) 09 April 2021 (2021-04-09) claims 1-71, and description, paragraphs 0005-0011, 0222-0225 and 0228 | 8-14 |
| X | WO 2020108530 A1 (INNOVENT BIOLOGICS (SUZHOU) CO., LTD.) 04 June 2020 (2020-06-04) description, page 21, paragraph 2 to page 22, table 3, and claims 1-23 | 1-7, 9-17 |

| ☑ Further documents are listed in the continuation of Box C. | ☑ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **16 July 2024** | **02 August 2024** |

| Name and mailing address of the ISA/CN | Authorized officer |
| --- | --- |
| **China National Intellectual Property Administration (ISA/ CN) China No. 6, Xitucheng Road, Jimenqiao, Haidian District, Beijing 100088** | |
| | Telephone No. |

Form PCT/ISA/210 (second sheet) (July 2022)

**EP 4 703 379 A1**

## INTERNATIONAL SEARCH REPORT



International application No.

**PCT/CN2024/090113**

### C. DOCUMENTS CONSIDERED TO BE RELEVANT

| Category* | Citation of document, with indication, where appropriate, of the relevant passages | Relevant to claim No. |
|---|---|---|
| Y | WO 2020108530 A1 (INNOVENT BIOLOGICS (SUZHOU) CO., LTD.) 04 June 2020 (2020-06-04) description, page 21, paragraph 2 to page 22, table 3, and claims 1-23 | 8-14 |
| Y | US 2018134784 A1 (JANSSEN BIOTECH, INC.) 17 May 2018 (2018-05-17) claims 1-18, and description, paragraphs 0010-0014 and 0190-0229 | 8-14 |
| Y | US 2014178401 A1 (GERALD HENRY NABOZNY et al.) 26 June 2014 (2014-06-26) claims 1-28 | 8-14 |
| A | WO 2011032148 A1 (ABBOTT LABORATORIES et al.) 17 March 2011 (2011-03-17) claims 1-187 | 1-17 |

Form PCT/ISA/210 (second sheet) (July 2022)

23

## INTERNATIONAL SEARCH REPORT

International application No.

**PCT/CN2024/090113**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

## INTERNATIONAL SEARCH REPORT

| International application No. |
| --- |
| **PCT/CN2024/090113** |

| Box No. II      Observations where certain claims were found unsearchable (Continuation of item 2 of first sheet) |
| --- |

This international search report has not been established in respect of certain claims under Article 17(2)(a) for the following reasons:

1. ☑ Claims Nos.: **1-14**
because they relate to subject matter not required to be searched by this Authority, namely:

Claims 1-14 relate to a method for treating a disease, which falls within methods for treatment of diseases as defined in PCT Rule 39.1(iv). However, a search is still performed on the basis of the pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2024/090113**

| Patent document cited in search report | | | Publication date (day/month/year) | Patent family member(s) | | | Publication date (day/month/year) |
|---|---|---|---|---|---|---|---|
| WO | 2021228113 | A1 | 18 November 2021 | CA | 3178853 | A1 | 18 November 2021 |
| | | | | KR | 20230009897 | A | 17 January 2023 |
| | | | | JP | 2023525825 | A | 19 June 2023 |
| | | | | US | 2023173069 | A1 | 08 June 2023 |
| | | | | TW | 202200203 | A | 01 January 2022 |
| | | | | TWI | 802882 | B | 21 May 2023 |
| | | | | EP | 4151233 | A1 | 22 March 2023 |
| | | | | EP | 4151233 | A4 | 19 June 2024 |
| | | | | BR | 112022022919 | A2 | 20 December 2022 |
| | | | | AU | 2021272212 | A1 | 08 December 2022 |
| CN | 112638420 | A | 09 April 2021 | US | 2022064280 | A1 | 03 March 2022 |
| | | | | CA | 3112579 | A1 | 19 March 2020 |
| | | | | AU | 2019337530 | A1 | 18 March 2021 |
| | | | | AU | 2019337530 | B2 | 10 August 2023 |
| | | | | JP | 2022500498 | A | 04 January 2022 |
| | | | | JP | 7203988 | B2 | 13 January 2023 |
| | | | | BR | 112021003209 | A2 | 11 May 2021 |
| | | | | WO | 2020055651 | A1 | 19 March 2020 |
| | | | | SG | 11202102240 | YA | 29 April 2021 |
| | | | | EP | 3849603 | A1 | 21 July 2021 |
| | | | | MX | 2021002647 | A | 14 September 2021 |
| | | | | JP | 2023036875 | A | 14 March 2023 |
| | | | | TW | 202023615 | A | 01 July 2020 |
| | | | | TWI | 725532 | B | 21 April 2021 |
| | | | | MA | 53602 | A | 15 December 2021 |
| | | | | TW | 202134274 | A | 16 September 2021 |
| | | | | TWI | 808397 | B | 11 July 2023 |
| | | | | EA | 202190504 | A1 | 10 June 2021 |
| | | | | KR | 20210042138 | A | 16 April 2021 |
| | | | | KR | 20230141933 | A | 10 October 2023 |
| | | | | IL | 281284 | A | 29 April 2021 |
| WO | 2020108530 | A1 | 04 June 2020 | JP | 2022505001 | A | 14 January 2022 |
| | | | | AU | 2019386021 | A1 | 17 December 2020 |
| | | | | AU | 2019386021 | B2 | 15 February 2024 |
| | | | | KR | 20210096559 | A | 05 August 2021 |
| | | | | EP | 3904382 | A1 | 03 November 2021 |
| | | | | EP | 3904382 | A4 | 13 July 2022 |
| | | | | US | 2021115130 | A1 | 22 April 2021 |
| | | | | TW | 202027791 | A | 01 August 2020 |
| | | | | TWI | 779253 | B | 01 October 2022 |
| | | | | CA | 3101670 | A1 | 04 June 2020 |
| | | | | CA | 3101670 | C | 06 February 2024 |
| | | | | AU | 2024200493 | A1 | 22 February 2024 |
| US | 2018134784 | A1 | 17 May 2018 | US | 11208474 | B2 | 28 December 2021 |
| | | | | US | 2021179703 | A1 | 17 June 2021 |
| | | | | AU | 2017362222 | A1 | 30 May 2019 |
| | | | | IL | 266607 | A | 31 July 2019 |
| | | | | KR | 20240065333 | A | 14 May 2024 |
| | | | | CA | 3044244 | A1 | 24 May 2018 |
| | | | | KR | 20190078648 | A | 04 July 2019 |

Form PCT/ISA/210 (patent family annex) (July 2022)

## INTERNATIONAL SEARCH REPORT
### Information on patent family members

| International application No. |
|---|
| **PCT/CN2024/090113** |

| Patent document cited in search report | | | Publication date (day/month/year) | Patent family member(s) | | | Publication date (day/month/year) |
|---|---|---|---|---|---|---|---|
| | | | | JP | 2023025173 | A | 21 February 2023 |
| | | | | MA | 46861 | A | 25 September 2019 |
| | | | | MX | 2019005661 | A | 07 October 2019 |
| | | | | WO | 2018093841 | A1 | 24 May 2018 |
| | | | | JP | 2020502261 | A | 23 January 2020 |
| | | | | EP | 3541422 | A1 | 25 September 2019 |
| | | | | EP | 3541422 | A4 | 06 May 2020 |
| US | 2014178401 | A1 | 26 June 2014 | US | 11078265 | B2 | 03 August 2021 |
| | | | | NZ | 700802 | A | 30 June 2017 |
| | | | | AR | 090923 | A1 | 17 December 2014 |
| | | | | EP | 2844284 | A1 | 11 March 2015 |
| | | | | MX | 2014013149 | A | 19 January 2015 |
| | | | | MX | 368653 | B | 10 October 2019 |
| | | | | PL | 3326649 | T3 | 25 April 2022 |
| | | | | TW | 201840335 | A | 16 November 2018 |
| | | | | TWI | 683669 | B | 01 February 2020 |
| | | | | PH | 12014502406 | A1 | 12 January 2015 |
| | | | | JP | 2015517465 | A | 22 June 2015 |
| | | | | JP | 6293120 | B2 | 14 March 2018 |
| | | | | CL | 2014002954 | A1 | 06 February 2015 |
| | | | | WO | 2013165791 | A1 | 07 November 2013 |
| | | | | EP | 4039275 | A1 | 10 August 2022 |
| | | | | AU | 2013256724 | A1 | 30 October 2014 |
| | | | | ES | 2908474 | T3 | 29 April 2022 |
| | | | | EP | 3326649 | A1 | 30 May 2018 |
| | | | | EP | 3326649 | B1 | 09 February 2022 |
| | | | | UY | 34779 | A | 29 November 2013 |
| | | | | CA | 2871985 | A1 | 07 November 2013 |
| | | | | CA | 2871985 | C | 10 October 2023 |
| | | | | KR | 20150013651 | A | 05 February 2015 |
| | | | | KR | 102124758 | B1 | 19 June 2020 |
| | | | | AU | 2018200239 | A1 | 01 February 2018 |
| | | | | AU | 2018200239 | B2 | 17 October 2019 |
| | | | | BR | 112014027372 | A2 | 08 August 2017 |
| | | | | TW | 201406391 | A | 16 February 2014 |
| | | | | TWI | 631957 | B | 11 August 2018 |
| | | | | EA | 201401204 | A1 | 29 May 2015 |
| | | | | IL | 264558 | A | 28 February 2019 |
| | | | | US | 2021317201 | A1 | 14 October 2021 |
| | | | | IL | 235059 | A0 | 31 December 2014 |
| | | | | IL | 258940 | A | 28 June 2018 |
| | | | | IL | 258940 | B | 30 May 2019 |
| WO | 2011032148 | A1 | 17 March 2011 | EP | 2477654 | A1 | 25 July 2012 |
| | | | | EP | 2477654 | A4 | 23 January 2013 |
| | | | | IL | 218633 | A0 | 31 May 2012 |
| | | | | US | 2014079714 | A1 | 20 March 2014 |
| | | | | SG | 179135 | A1 | 30 May 2012 |
| | | | | JP | 2013504598 | A | 07 February 2013 |
| | | | | NZ | 598722 | A | 30 May 2014 |
| | | | | RU | 2012114854 | A | 27 October 2013 |

Form PCT/ISA/210 (patent family annex) (July 2022)

**INTERNATIONAL SEARCH REPORT**
Information on patent family members

International application No.

**PCT/CN2024/090113**

| Patent document cited in search report | Publication date (day/month/year) | Patent family member(s) | | Publication date (day/month/year) |
|---|---|---|---|---|
| | | KR 20120112384 | A | 11 October 2012 |
| | | TW 201127400 | A | 16 August 2011 |
| | | MX 2012003138 | A | 04 July 2012 |
| | | US 2011206680 | A1 | 25 August 2011 |
| | | US 8557239 | B2 | 15 October 2013 |
| | | CA 2773556 | A1 | 17 March 2011 |
| | | IN DEN2012 | A | 21 August 2015 |
| | | AU 2010291927 | A1 | 12 April 2012 |
| | | KR 20140048229 | A | 23 April 2014 |

Form PCT/ISA/210 (patent family annex) (July 2022)

## REFERENCES CITED IN THE DESCRIPTION

### Patent documents cited in the description

- CN 2019121261 W
- WO 2020108530 A1
- CN 2021093219 W
- WO 2021228113 A1

### Non-patent literature cited in the description

- **OPPMANN et al.** *Immunity*, 2000, vol. 13, 715
- **BRAVO** ; **HEATH**. *EMBO J.*, 2000, vol. 19, 2399-2411
- **CHOTHIA et al.** *Nature*, 1989, vol. 342, 877-883
- **AL-LAZIKANI et al.** Standard conformations for the canonical structures of immunoglobulins. *Journal of Molecular Biology*, 1997, vol. 273, 927-948
- **KABAT et al.** Sequences of Proteins of Immunological Interest. U.S. Department of Health and Human Services, National Institutes of Health, 1987
- **KABAT et al.** Sequences of Proteins of Immunological Interest. Public Health Service, National Institutes of Health, 1991
- **KENNETH B GORDON**. Efficacy and safety of risankizumab in moderate-to-severe plaque psoriasis (UltIMMa-1 and UltIMMa-2): results from two double-blind, randomised, placebo-controlled and ustekinumab-controlled phase 3 trials. *Lancet*, 25 August 2018, vol. 392 (10148), 650-661